# EUROPÄISCHE PATENTSCHRIFT

(11) **EP 0 766 661 B1**
(45) Veröffentlichungstag und Bekanntmachung des Hinweises auf die Patenterteilung: **25.08.1999**
(21) Anmeldenummer: 95922507.9
(22) Anmeldetag: 06.06.1995
(51) Int. Cl.: C07C 69/675, A61K 7/50, A61K 7/48, A61K 7/06, A61K 47/14, C07C 67/08

(54) **POLYOLPOLYHYDROXYSTEARATE**
POLYOLPOLYHYDROXYSTEARATES
POLYOLPOLYHYDROXYSTEARATES

(30) Priorität: 13.06.1994 DE G0442051 U
(43) Veröffentlichungstag der Anmeldung: 09.04.1997
(73) Patentinhaber: Henkel Kommanditgesellschaft auf Aktien, 40191 Düsseldorf (DE)
(72) Erfinder: ANSMANN, Achim, D-40699 Erkrath (DE); KAWA, Rolf, D-40789 Monheim (DE); VON KRIES, Rainer, D-89257 Illertissen (DE); STRAUSS, Gabriele, D-40589 Düsseldorf (DE)
(86) Internationale Anmeldenummer: EP9502146
(87) Internationale Veröffentlichungsnummer: WO9534528

(56) Entgegenhaltungen:
- GB-A- 1 524 782
- DATABASE WPI Week 9233 Derwent Publications Ltd., London, GB; AN 92-272108 & JP,A,04 178 316 (KAO CORP) , 25.Juni 1992
- PATENT ABSTRACTS OF JAPAN vol. 16 no. 485 (C-0993) ,8.Oktober 1992 & JP,A,04 178316 (KAO CORP) 25.Juni 1992,

## Beschreibung

### Gebiet der Erfindung

Die Erfindung betrifft neue Polyglycerinpolyhydroxystearate, erhältlich durch Veresterung von Polyhydroxystearinsäure mit technischem Polyglycerin einer definierten Zusammensetzung, ein Verfahren zu ihrer Herstellung, sowie die Verwendung der neuen Polyglycerinpolyhydroxystearate als W/O-Emulgatoren.

### Stand der Technik

Polyglycerinpolyricinoleate sind seit langem als W/O-Emulgatoren bekannt und können zur Formulierung von niedrigviskosen W/O-Emulsionen eingesetzt werden [vgl. **EP 0559013 A1** (Th. Gold-schmidt), **EP 0440203 A1** (Lotte Co.) und **WO 85/04346** (Meiji Milk Prods.)]. Es zeigt sich jedoch, daß Polyglycerinpolyrinoleate des Marktes nicht mit allen in der Kosmetik üblicherweise eingesetzten Öle Emulsionen bilden, sondern nur mit solchen eines bestimmten Polaritätsbereiches; zudem sind diese Emulsionen nur eingeschränkt lagerstabil. Ein wesentlicher Nachteil besteht vor allem darin, daß die handelsüblichen Produkte nicht in der Lage sind, Emulsionen mit stark polaren Ölen wie beispielsweise Pflanzenölen ausreichend zu stabilisieren. Im Hinblick auf die besondere ökotoxikologische Verträglichkeit derartiger Emulsionen wird jedoch gerade dies im Markt gewünscht.

In diesem Zusammenhang sei auf die **GB 1524782 A1** hingewiesen, welche ein Verfahren zur Herstellung von Polyolpolyhydroxystearaten offenbart, bei dem man Polyhydroxystearinsäure mit einem Kondensationsgrad von 2 bis 10 beispielsweise mit Glycerin verestert. Aus **Data Base WPI, Week 9233, AN 92-272108** und **Patent Abstract of Japan, Vol. 16, No. 485 (C-0993)** sind zudem kosmetische Zubereitungen bekannt, die Glycerinester der Polyhydroxystearinsäure und Polyricinolsäure enthalten.

Die Aufgabe der Erfindung hat nun darin bestanden, neue W/O- Emulgatoren zur Verfügung zu stellen, die mit einem breiten Spektrum von Ölkörpern lagerstabile Emulsionen ergeben.

### Beschreibung der Erfindung

Gegenstand der Erfindung sind Polyglycerinpolyhydroxystearate, die man erhält, indem man Polyhydroxystearinsäure mit einem Eigenkondensationsgrad im Bereich von 2 bis 20, vorzugsweise 2 bis 10, mit Polyglycerinen der Zusammensetzung

| | |
|---|---|
| Glycerin | 5 bis 30 Gew.-% |
| Diglycerine | 15 bis 40 Gew.-% |
| Triglycerine | 10 bis 30 Gew.-% |
| Tetraglycerine | 5 bis 20 Gew.-% |
| Pentaglycerine | 2 bis 10 Gew.-% |
| Oligoglycerine | ad 100 Gew.-% |

in an sich bekannter Weise verestert.

Überraschenderweise hat sich gezeigt, daß Kondensationsprodukte von technischem Polyglycerin mit Polyhydroxystearinsäure deutlich bessere Emulgiereigenschaften aufweisen als vergleichbare bekannte Produkte auf Basis von Polyricinolsäure. Insbesondere können auch stark polare Pflanzenöle in stabile Emulsionen eingebracht werden. Die Erfindung schließt ferner die Erkenntnis ein, daß bereits die Zugabe geringer Mengen der erfindungsgemäßen Polyglycerinpolyhydroxystearate zu Polyolpolyricinoleaten deren Emulgiereigenschaften nachhaltig verbessert. Ferner wurde überraschenderweise festgestellt, daß die Kondensationsprodukte auf Basis der 12-Hydroxystearinsäure flüssig sind, obwohl die eingesetzte Säure einen Schmelzpunkt im Bereich von 75°C aufweist. Somit ist neben der klassischen "Heiß-Heiß-Herstellung auch eine energiesparende "Kalt-Kalt"-Herstellung der Emulsionen problemlos möglich.

In einer bevorzugten Ausführungsform betrifft die Erfindung Polyglycerinpolyhydroxystearate, die man erhält, indem man Polyhydroxystearinsäure mit einem Eigenkondensationsgrad im Bereich von 2 bis 20, vorzugsweise 2 bis 10, mit einem Polyglyceringemisch der Zusammensetzung (GC-Methode)

| | |
|---|---|
| Glycerine | 15 bis 30 Gew.-% |
| Diglycerine | 20 bis 32 Gew.-% |
| Triglycerine | 15 bis 25 Gew.-% |
| Tetraglycerine | 8 bis 15 Gew.-% |
| Pentaglycerine | 3 bis 8 Gew.-% |
| Oligoglycerine | ad 100 Gew.-% |

in an sich bekannter Weise verestert.

Ein weiterer Gegenstand der Erfindung betrifft ein Verfahren zur Herstellung von Polyglycennpolyhydroxystearaten, bei dem man Polyhydroxystearinsäure mit einem Eigenkondensationsgrad im Bereich von 2 bis 20 mit Polyglycerin der Zusammensetzung

| | |
|---|---|
| Glycerin | 5 bis 30 Gew.-% |
| Diglycerine | 15 bis 40 Gew.-% |
| Triglycerine | 10 bis 30 Gew.-% |
| Tetraglycerine | 5 bis 20 Gew.-% |
| Pentaglycerine | 2 bis 10 Gew.-% |
| Oligoglycerine | ad 100 Gew.-% |

in an sich bekannter Weise verestert.

### Herstellung der Polyglcerinpolyhydroxystearate

Die Herstellung der Polyolpolyhydroxystearate kann in an sich bekannter Weise erfolgen, wobei man vorzugsweise zunächst das Polyglycerin und dann die Polyhydroxystearinsäure hergestellt und schließlich beide verestert. Die Herstellung eines Polyglycerins der oben genannten Zusammensetzung kann durch Eigenkondensation von Glycerin in Gegenwart von geeigneten Katalysatoren wie beispielsweise Kaliumcarbonat, Silicaten gemäß **DE 4029323 A1** (Henkel) oder Boraten gemäß **DE 4117033 A1** (Henkel) bei Temperaturen im Bereich von 200 bis 260°C durchgeführt werden. Die Herstellung der Polyhydroxystearinsäure erfolgt beispielsweise durch alkalisch katalysierte Polykondensation von Hydroxystearinsäure, vorzugsweise 12-Hydroxystearinsäure, die durch Härtung von Ricinolsäure bzw. technischer Ricinusölfettsäure gewonnen wird. Vorzugsweise werden dabei lineare Veresterungsprodukte mit 2 bis 10 und insbesondere 2 bis 8 Fettsäureeinheiten gebildet. Typischerweise wird die folgende Verteilung (GPC-Methode) erreicht:

| | |
|---|---|
| Monomere | 1 bis 10 Gew.-% |
| Dimere | 5 bis 15 Gew.-% |
| Trimere | 5 bis 15 Gew.-% |
| Tetramere | 5 bis 15 Gew.-% |
| Pentamere | 5 bis 15 Gew.-% |
| Hexamere | 5 bis 15 Gew.-% |
| Heptamere | 5 bis 15 Gew.-% |
| Octamere | 1 bis 10 Gew.-% |
| Oligomere | ad 100 Gew.-% |

In einer besonderen Ausführungsform der Erfindung werden Gemische von Hydroxystearinsäure und Ricinolsäure bzw. technischer Ricinusölfettsäure, die zu etwa 90 Gew.-% aus Ricinolsäure besteht, im Gewichtsverhältnis 99 : 1 bis 1 : 99 und vorzugsweise 75 : 25 bis 10 : 90 eingesetzt. In gleicher Weise ist es möglich, die Säuren einzeln zu kondensieren und anschließend die Kondensate abzumischen. Bei der nachfolgenden Kondensation des Polyglycerins mit der Polyhydroxystearinsäure bzw. den Gemischen mit Polyricinolsäure, wird eine komplexe Mischung homologer Polyester gebildet. Die Anteile an Mono-, Di-, Tri- und Oligoestern in den erfindungsgemäßen Polyglycerinpolyhydroxystearaten richtet sich nach den Einsatzverhältnissen der Ausgangsverbindungen. In einer bevorzugten Ausführungsform des erfindungsgemäßen Verfahrens wird ein Polyglycerinpolyhydroxystearat mit besonders vorteilhaften anwendungstechnischen Eigenschaften erhalten, indem man etwa 1000 kg 12-Hydroxystearinsäure solange einer Eigenkondensation unterwirft, bis ein Produkt mit einer Säurezahl im Bereich von 50 bis 55 resultiert und dieses dann mit etwa 150 kg Polyglycerin der oben angegebenen Zusammensetzung weiter verestert, bis die Säurezahl bis auf einen Wert kleiner 2 abgenommen hat. Kondensationsprodukte auf Basis von Polyglycerin und Polyhydroxystearinsäure bzw. Polyhydroxystearinsäure/Polyricinolsäure können über ihre lodzahl charakterisiert werden. Typische Beispiele sind Polyester mit einer lodzahl < 10 (Basis 100 % 12-Hydroxystearinsäure) bzw. 65 bis 80 (Basis 90 % 12-Hydroxystearinsäure, 10 % Ricinolsäure).

### Kosmetische und pharmazeutische Zubereitungen

Die Polyglycerinpolyhydroxystearate eignen sich alleine oder im Gemisch mit Polyglycerinpolyricinoleaten im Gewichtsverhältnis 99 : 1 bis 1 : 99, vorzugsweise 75 : 25 bis 10 : 90 zur Herstellung von kosmetischen und/oder pharmazeutischen Zubereitungen. Als Hilfs- und Zusatzstoffe können diese Mittel ferner Ölkörper, Co-Emulgatoren, Fette und Wachse, Stabilisatoren, Verdickungsmittel, biogene Wirkstoffe, Filmbildner, Duftstoffe, Farbstoffe, Perlglanzmittel, Konservierungsmittel, UV-Filter, Pigmente, Elektrolyte (z.B. Magnesiumsulfat) und pH-Regulatoren enthalten.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₀-Fettsäuren mit linearen C₆-C₂₀-Fettalkoholen, Ester von verzweigten C₆-C₁₃-Carbonsäuren mit linearen C₁₆-C₁₈-Fettalkoholen, Ester von linearen C₁₀-C₁₈-Fettsäuren mit verzweigten Alkoholen, insbesondere 2-Ethylhexanol, Ester von linearen und/oder verzweigten Fettsäuren mit zweiwertigen Alkoholen und/oder Guerbetalkoholen, Triglyceride auf Basis C₆-C₁₀-Fettsäuren, pflanzliche Öle, verzweigte primäre Alkohole, substituierte Cyclohexane, Guerbetcarbonate und/oder Dialkylether in Betracht. Von besonderer Bedeutung ist hierbei, daß sich die erfindungsgemäßen Polyglycerinpolyricinoleate zur Bildung von Emulsionen unter Verwendung sowohl von polaren, als auch mittel- oder unpolaren Olkörpern mit Dipolmomenten in einem Bereich von kleiner 1 bis größer 2,5 Debye eignen.

Als **Co-Emulgatoren** können nichtionogene, ampholytische und/oder zwitterionische grenzflächenaktive Verbindungen verwendet werden, die sich durch eine lipophile, bevorzugt lineare, Alkyl- oder Alkenylgruppe und mindestens eine hydrophile Gruppe auszeichnen. Diese hydrophile Gruppe kann sowohl eine ionogene als auch eine nichtionogene Gruppe sein. Nichtionogene Emulgatoren enthalten als hydrophile Gruppe z. B. eine Polyolgruppe, eine Polyalkylenglycolethergruppe oder eine Kombination aus Polyol- und Polyglycolethergruppe. Bevorzugt sind solche Mittel, die als **O/W-Emulgatoren** nichtionogene Tenside aus der Gruppe der
(a1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C- Atomen und an Alkylphenole mit 8 bis 15 C-Atomen in der Alkylgruppe,
(a2) C₁₂-C₁₈-Fettsäuremono- und -diester von Anlagerungsprodukten von 1 bis 30 Mol Ethylenoxid an Glycerin,
(a3) Glycerinmono- und -diester und Sorbitanmono- und -diester von gesättigten und ungesättigten C₈-C₁₈-Fettsäuren und deren Ethylenoxidanlagerungsprodukte,
(a4) C₈-C₁₈-Alkylmono- und -oligoglycoside und deren ethoxylierte Analoga und
(a5) Anlagerungsprodukte von 15 bis 60 Mol Ethylenoxid an Ricinusöl und/oder gehärtetes Ricinusöl,

enthalten. Ebenfalls geeignet sind Mischungen von Verbindungen aus mehreren dieser Substanzklassen. Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C₁₂-C₁₈-Fettsäuremono- und -diester von Anlagerungsprodukten von Ethylenoxid an Glycerin sind aus **DE 2024051 PS** als Rückfettungsmittel für kosmetische Zubereitungen bekannt. C₈-C₁₈-Alkylmono- und oligoglycoside, ihre Herstellung und ihre Verwendung als oberflächenaktive Stoffe sind beispielsweise aus **US 3839318, US 3707535, US 3547828, DE 1943689 OS, DE 2036472 OS** und **DE 3001064 A1** sowie **EP 0077167** Al bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt. Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine -COO⁻- oder -SO₃⁻-Gruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 Kohlenstoffatomen in der Alkyl- oder Acylgruppe sowie das Kokosacylaminoethylhydroxyethylcarboxymethylglycinat. Besonders bevorzugt ist das unter der CTFA-Bezeichnung Cocamidopropyl Betaine bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C₈-C₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂-C₁₈-Acylsarcosin.

Als **W/O-Emulgatoren** kommen in Betracht:
(b1) Anlagerungsprodukte von 2 bis 15 Mol Ethylenoxid and Ricinusöl und/oder gehärtetes Ricinusöl;
(b2) Partialester auf Basis linearer, verzweigter, ungesättigter bzw. gesättigter C₁₂-C₂₂-Fettsäuren, Ricinolsäure sowie 12-Hydroxystearinsäure und Glycerin, Polyglycerin, Pentaerythrit, Dipentaerythrit, Zuckeralkohole (z.B. Sorbit) sowie Polyglucoside (z.B. Cellulose);
(b3) Trialkylphosphate;
(b4) Wollwachsalkohole;
(b5) Polysiloxan-Polyalkyl-Polyether-Copolymere bzw. entsprechende Derivate;
(b6) Mischester aus Pentaerythrit, Fettsäuren, Citronensäure und Fettalkohol gemäß **DE 1165574 PS** sowie
(b7) Polyalkylenglycole.

### Weitere Zusatzstoffe

Typische Beispiele für **Fette** sind Glyceride, als **Wachse** kommen u.a. Bienenwachs, Paraffinwachs oder Mikrowachse gegebenenfalls in Kombination mit hydrophilen Wachsen wie beispielsweise Fettalkohole, Monoglyceride und Fettsäuren in Frage. Als **Stabilisatoren** können Metallsalze von Fettsäuren wie z.B. Magnesium-, Aluminium- und/oder Zinkstearat eingesetzt werden. Geeignete **Verdickungsmittel** sind beispielsweise vernetzte Polyacrylsäuren und deren Derivate, Polysaccharide, insbesondere Xanthan-Gum, Guar-Guar, Agar-Agar, Alginate und Tylosen, Carboxymethylcellulose und Hydroxyethylcellulose, Polyacrylate, Polyvinylalkohol und Polyvinylpyrrolidon. Unter **biogenen Wirkstoffen** sind beispielsweise Pflanzenextrakte, Eiweißhydrolysate und Vitaminkomplexe zu verstehen. Gebräuchliche Filmbildner sind beispielsweise Hydrocolloide wie Chitosan, mikrokristallines Chitosan oder quaterniertes Chitosan, Polyvinylpyrrolidon, Vinylpyrrolidon-Vinylacetat-Copolymerisate, Polymere der Acrylsäurereihe, quaternäre Cellulose-Derivate und ähnliche Verbindungen. Als **Konservierungsmittel** eignen sich beispielsweise Formaldehydlösung, p-Hydroxybenzoat oder Sorbinsäure. Als **Perlglanzmittel** kommen beispielsweise Glycoldistearinsäureester wie Ethylenglycoldistearat, aber auch Fettsäuren und Fettsäuremonoglycolester in Betracht. Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, veröffentlicht im Verlag Chemie, Weinheim, 1984**, zusammengestellt sind. Diese Farbstoffe werden üblicherweise in Konzentrationen von 0,001 bis 0,1 Gew.-%, bezogen auf die gesamte Mischung, eingesetzt. Der Gesamtanteil der Hilfs- und Zusatzstoffe kann 0,01 bis 80, vorzugsweise 0,05 bis 40 Gew.-% und der nicht wäßrige Anteil ("Aktivsubstanz") 20 bis 80, vorzugsweise 30 bis 70 Gew.-% - bezogen auf die Mittel - betragen. Die Herstellung der Mittel kann in an sich bekannter Weise, d.h. beispielsweise durch Heiß-, Kalt-, Heiß-Heiß/Kalt- bzw. PIT-Emulgierung erfolgen. Hierbei handelt es sich um ein rein mechanisches Verfahren, eine chemische Reaktion findet nicht statt.

### Gewerbliche Anwendbarkeit

Die erfindungsgemäßen Polyglycerinpolyhydroxystearate zeichnen sich durch ein verbessertes Emulgiervermögen aus. Die resultierenden Emulsionen besitzen eine gegenüber den Produkten des Stands der Technik höhere Lager- und insbesondere Wärmestabilität. Ein weiterer Gegenstand der Erfindung betrifft daher die Verwendung der erfindungsgemäßen Polyglycerinpolyhydroxystearate, gegebenenfalls in Abmischung mit Polyglycerinpolyricinoleaten, als W/O-Emulgatoren für kosmetische und/oder pharmazeutische Zubereitungen wie z.B. Hautcremes, Körperlotionen, Sonnenschutzmittel und dergleichen, in denen sie in Konzentrationen von 1 bis 20, vorzugsweise 2 bis 10 Gew.-% - bezogen auf die Mittel - enthalten sein können.

### Beispiele

**Viskosität und Stabilität**. Die Eigenschaften der erfindungsgemäßen Emulgatoren sowie des Vergleichsproduktes wurden in verschiedenen W/O-Emulsionen gemäß Tabelle 1 getestet. Die Zusammensetzung der Polyglycerinkomponenten betrug 20 Gew.-% Glycerin, 30 Gew.-% Diglycerine, 20 Gew.-% Triglycerine, 15 Gew.-% Tetraglycerine, 5 Gew.-% Pentaglycerine und 10 Gew.-% Oligoglycerine. Die Zusammensetzung der Polyhydroxyfettsäure betrug 5 Gew.-% Monomere, jeweils 10 Gew.-% Dimere bis Heptamere, 6 Gew.-% Octamere und ad 100 Gew.-% Oligomere. Die Beispiele 1 bis 3 sind erfindungsgemäß, Beispiel V1 dient zum Vergleich. Die Viskosität der Produkte wurde nach Lagerung (1 Tag, 1 Woche bzw. 4 Wochen) nach Brookfield, RVF, 23°C, Spindel 5, 10 UpM bestimmt. Die Ergebnisse sind in Tabelle 1 zusammengestellt.

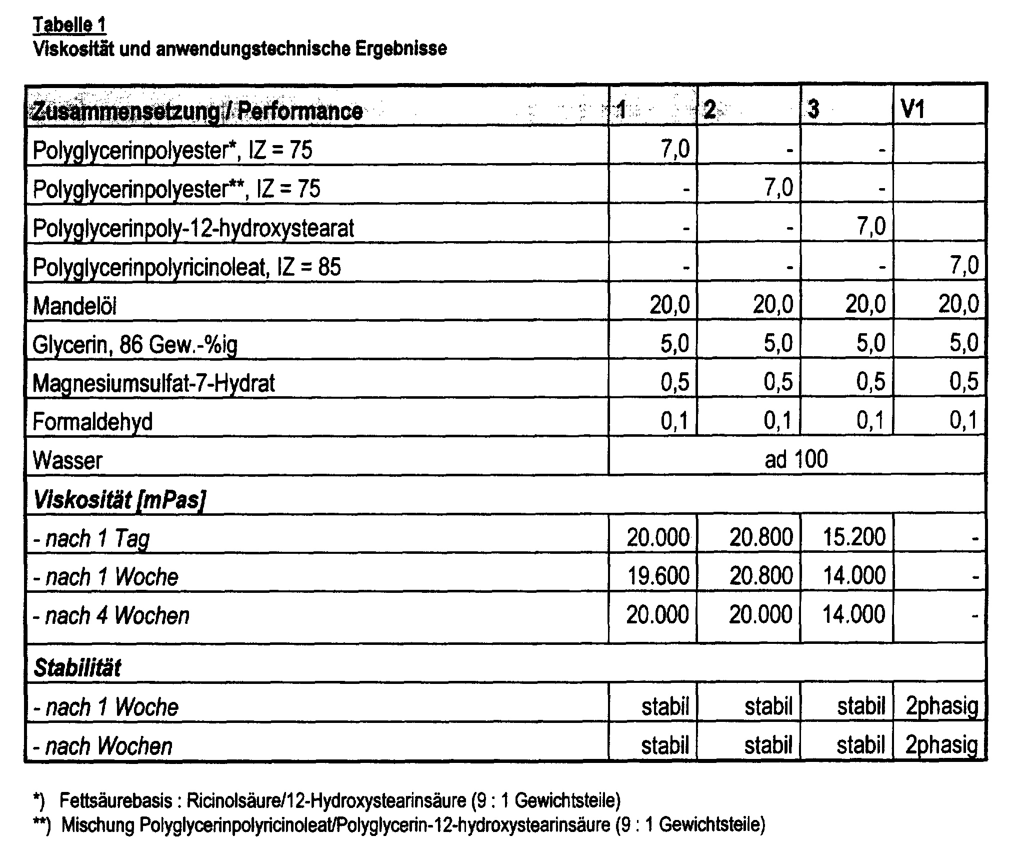

**Formulierungsbeispiele**. In Tabelle 2 sind Formulierungsbeispiele für eine kosmetische Hautlotion (4) sowie für ein Nährcreme (5) angegeben. Die Viskositäten der beiden Produkte wurden mit einem Brookfield RVF-Viskosimeter bei 23°C bestimmt. Für das Produkt (4) wurde Spindel 5 (10 UpM) und für Produkt (5) Spindel E (4 UpM, mit Helipath) verwendet.

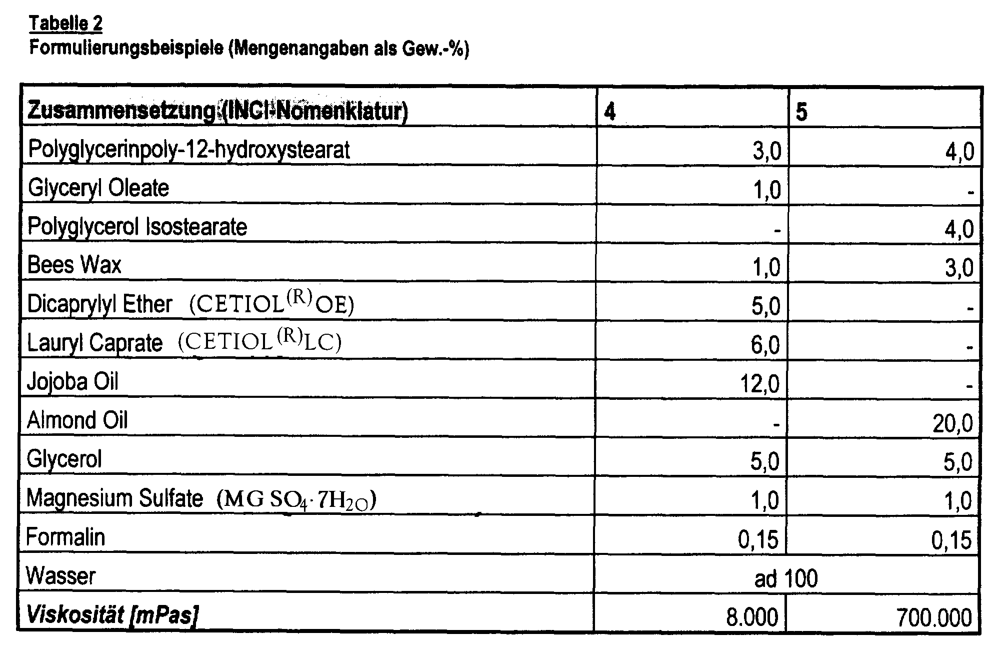

## Patentansprüche

1. Polyglycerinpolyhydroxystearate, dadurch erhältlich, daß man Polyhydroxystearinsäure mit einem Eigenkondensationsgrad im Bereich von 2 bis 20 mit Polyglycerinen der Zusammensetzung
| | |
|---|---|
| Glycerin | 5 bis 30 Gew.-% |
| Diglycerine | 15 bis 40 Gew.-% |
| Triglycerine | 10 bis 30 Gew.-% |
| Tetraglycerine | 5 bis 20 Gew.-% |
| Pentaglycerine | 2 bis 10 Gew.-% |
| Oligoglycerine | ad 100 Gew.-% |
in an sich bekannter Weise verestert.

2. Verfahren zur Herstellung von Polyglycerinpolyhydroxystearaten, bei dem man Polyhydroxystearinsäure mit einem Eigenkondensationsgrad im Bereich von 2 bis 20 mit Polyglycerin der Zusammensetzung
| | |
|---|---|
| Glycerin | 5 bis 30 Gew.-% |
| Diglycerine | 15 bis 40 Gew.-% |
| Triglycerine | 10 bis 30 Gew.-% |
| Tetraglycerine | 5 bis 20 Gew.-% |
| Pentaglycerine | 2 bis 10 Gew.-% |
| Oligoglycerine | ad 100 Gew.-% |
in an sich bekannter Weise verestert.

3. Verwendung von Polyglycerinpolyhydroxystearaten nach Anspruch 1 als W/O-Emulgatoren für kosmetische und/oder pharmazeutische Zubereitungen.

## Claims

1. Polyglycerol polyhydroxystearates obtainable by esterifying polyhydroxystearic acid having a degree of self-condensation of 2 to 20 with polyglycerols of the following composition:
| | |
|---|---|
| glycerol | 5 to 30% by weight |
| diglycerols | 15 to 40% by weight |
| triglycerols | 10 to 30% by weight |
| tetraglycerols | 5 to 20% by weight |
| pentaglycerols | 2 to 10% by weight |
| oligoglycerols | to 100 % by weight |
in known manner.

2. A process for the production of polyglycerol polyhydroxystearates, in which polyhydroxystearic acid having a degree of self-condensation of 2 to 20 is esterified with polyglycerol of the following composition:
| | |
|---|---|
| glycerol | 5 to 30% by weight |
| diglycerols | 15 to 40% by weight |
| triglycerols | 10 to 30% by weight |
| tetraglycerols | 5 to 20% by weight |
| pentaglycerols | 2 to 10% by weight |
| oligoglycerols | to 100 % by weight |
in known manner.

3. The use of the polyglycerol polyhydroxystearates claimed in claim 1 as w/o emulsifiers for cosmetic and/or pharmaceutical formulations.

## Revendications

1. Polyhydroxystéarates de polyglycéryle accessibles en ce qu'
on estérifie d'une manière connue en soi des acides polyhydroxystéariques ayant un degré d'autocondensation dans la zone de 2 à 20 avec des polyglycérols de composition :
| | |
|---|---|
| Glycérol | 5 à 30 % en poids |
| Diglycérols | 15 à 40 % en poids |
| Triglycérols | 10 à 30 % en poids |
| Tétraglycérols | 5 à 20 % en poids |
| Pentaglycérols | 2 à 10 % en poids |
| Oligoglycérols | qsp 100 % en poids. |

2. Procédé de préparation de polyhydroxystéarates de polyglycéryle dans lequel on estérifie des acides polyhydroxystéariques ayant un degré d'autocondensation dans la zone de 2 à 20, avec un polyglycérol de composition :
| | |
|---|---|
| Glycérol | 5 à 30 % en poids |
| Diglycérols | 15 à 40 % en poids |
| Triglycérols | 10 à 30 % en poids |
| Tétraglycérols | 5 à 20 % en poids |
| Pentaglycérols | 2 à 10 % en poids |
| Oligoglycérols | qsp 100 % en poids |
d'une manière connue en soi.

3. Utilisation de polyhydroxystéarates de polyglycéryle selon la revendication 1 comme agents émulsionnants W/O pour des préparations cosmétiques et/ou pharmaceutiques.
